(19) Europäisches Patentamt
European Patent Office
Office européen des brevets

(11) **EP 4 337 309 B1**

(12) **EUROPÄISCHE PATENTSCHRIFT**

(45) Veröffentlichungstag und Bekanntmachung des Hinweises auf die Patenterteilung:
**07.08.2024 Patentblatt 2024/32**

(21) Anmeldenummer: **22723457.2**

(22) Anmeldetag: **28.04.2022**

(51) Internationale Patentklassifikation (IPC):
**A61N 5/06** (2006.01)

(52) Gemeinsame Patentklassifikation (CPC):
**A61N 5/0614;** A61N 2005/0615; A61N 2005/0661

(86) Internationale Anmeldenummer:
**PCT/EP2022/061348**

(87) Internationale Veröffentlichungsnummer:
**WO 2022/238134 (17.11.2022 Gazette 2022/46)**

(54) **STRAHLUNGSQUELLE UND HAUTBESTRAHLUNGSGERÄT**

RADIATION SOURCE AND SKIN IRRADIATION DEVICE

SOURCE DE RAYONNEMENT ET DISPOSITIF D'IRRADIATION DE LA PEAU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.05.2021 EP 21173008**

(43) Veröffentlichungstag der Anmeldung:
**20.03.2024 Patentblatt 2024/12**

(73) Patentinhaber: **Schaffron, Günter**
**49716 Meppen (DE)**

(72) Erfinder: **SCHAFFRON, Günter**
**49716 Meppen (DE)**

(74) Vertreter: **Hauck Patentanwaltspartnerschaft mbB**
**Postfach 11 31 53**
**20431 Hamburg (DE)**

(56) Entgegenhaltungen:
**DE-A1- 19 611 763      FR-A1- 2 764 813**
**US-A1- 2013 172 963**

- **ANONYMOUS: "DIN 5031-10:2018-03 Strahlungsphysik im optischen Bereich und Lichttechnik - Teil 10: Photobiologisch wirksame Strahlung, Größen, Kurzzeichen und Wirkungsspektren", DIN NORM,, vol. 5031-10, 1 March 2018 (2018-03-01), pages 1 - 102, XP009545400**
- **VOLKMER BEATE ET AL: "Schutz des Menschen vor den Gefahren solarer UV- Strahlung und UV-Strahlung in Solarien", 11 February 2016 (2016-02-11), Bonn, pages 1 - 121, XP055852193, Retrieved from the Internet <URL:https://www.ssk.de/SharedDocs/Beratung sergebnisse_PDF/2016/2016-02-11_Empf_UV-Sc hutz%20BA.pdf?__blob=publicationFile> [retrieved on 20211018]**

**Beschreibung**

Die Erfindung betrifft eine Strahlungsquelle zur Abgabe von UV-Strahlung zur Bräunung der Haut sowie ein Hautbestrahlungsgerät umfassend eine solche Strahlungsquelle.

Als Strahlungsquellen für Hautbestrahlungsgeräte zur Bräunung der Haut, also für Solarien, werden üblicherweise UV-Niederdruckröhren oder UV-Hochdruckbrenner mit Filterscheiben eingesetzt. So können beispielsweise lediglich UV-Niederdruckröhren oder UV-Hochdruckbrenner als Bestrahlungsquellen vorgesehen sein. Auch kann eine Kombination aus UV-Niederdruckröhren und UV-Hochdruckbrennern mit Filterscheiben vorgesehen sein, wobei üblicherweise die Niederdruckröhren zur Bestrahlung des Körperbereichs und der Hochdruckbrenner zur Bestrahlung des Gesichtsbereichs verwendet werden. Auch können LED-Strahler für den Gesichtsbereich oder sogar zur Ganzkörperbestrahlung eingesetzt werden.

Strahlungsquellen zur Bräunung der Haut geben UV-Strahlung üblicherweise in einem Bereich zwischen 280 nm und 400 nm Wellenlänge oder in Teilbereichen davon ab. Strahlung im UVA-Bereich zwischen 315 nm und 400 nm Wellenlänge hat eine größere Eindringtiefe in biologisches Gewebe und trägt im Wesentlichen zur direkten Pigmentierung bei, also zu der Entwicklung einer kurzfristigen Bräune. UVB-Strahlung hingegen, im Wellenlängenbereich von 280 nm bis 315 nm, dringt weniger tief in biologisches Gewebe ein und bewirkt dadurch eine verzögerte Bildung von Melanin und damit eine indirekte Pigmentierung, also eine verzögerte, längerfristige Bräunung. Um ein optimales Bräunungsergebnis zu erhalten, ist ein abgestimmtes Verhältnis von UVA- zu UVB-Strahlung und eine definierte Bestrahlungsstärke von Bedeutung.

Die wellenlängenabhängigen Wirkungen von UV-Strahlung insbesondere auf die Haut sind Gegenstand umfangreicher Untersuchungen. Wichtige Definitionen finden sich zum Beispiel in der Norm DIN 5031-10:2018-03. Darin wird die auf die Haut auftreffende Strahlung durch ihre spektrale Bestrahlungsstärke $E_\lambda(\lambda)$ charakterisiert. Die spektrale Bestrahlungsstärke $E_\lambda(\lambda)$ gibt die in einem bestimmten Wellenlängenbereich auf die Haut auftreffende Strahlungsleistung an, in der Regel in Watt pro Quadratmeter. Sie entspricht der von einer Strahlungsquelle eintreffenden Strahlungsleistung.

Eine andere wichtige Kenngröße, mit der in der genannten Norm die biologischen Wirkungen der auf die Haut auftreffenden Strahlung beschrieben werden, ist die sogenannte photobiologisch wirksame Bestrahlungsstärke $E_{biol}$ (im Folgenden auch kurz bezeichnet als biologische Bestrahlungsstärke). In Kenntnis der relativen spektralen Empfindlichkeit $A_{biol}(\lambda)$ des jeweiligen biologischen Vorgangs kann sie wie folgt aus der spektralen Bestrahlungsstärke $E_\lambda(\lambda)$ berechnet werden:

$$E_{biol} = \int E_\lambda(\lambda) \, A_{biol}(\lambda) \, d\lambda$$

Die Integration ist über den gesamten relevanten Wellenlängenbereich auszuführen. Die relative spektrale Empfindlichkeit $A_{biol}(\lambda)$ wird auch als spektraler Wirkungsfaktor oder als Wirkungsspektrum bezeichnet.

Spektrale Wirkungsfaktoren sind für zahlreiche biologische Wirkungen der UV-Strahlung bekannt und zum Beispiel in der genannten Norm angegeben, so dass aus einer bekannten (z.B. mit einem Doppelmonochromator gemessenen) spektralen Bestrahlungsstärke $E_\lambda(\lambda)$ einer Strahlungsquelle die jeweilige biologische Bestrahlungsstärke berechnet werden kann. Dies gilt beispielsweise für die folgenden photobiologisch wirksamen Bestrahlungsstärken:

- die erythemwirksame Bestrahlungsstärke $E_{er}$. Sie ist für die Bildung des UV-Erythems verantwortlich. Ein UV-Erythem, im folgenden auch kurz als Erythem bezeichnet, ist das Symptom einer akuten Hautentzündung, die durch UV-Bestrahlung entsteht, auch bezeichnet als Sonnenbrand.

- die NMSC-wirksame Bestrahlungsstärke $E_{NMSC}$. Sie ist für die Bildung des weißen Hautkrebses verantwortlich. NMSC steht für *non melanoma skin cancer*.

- die sofortpigmentierungswirksame Bestrahlungsstärke $E_{pi}$. Sie ist für die direkte Pigmentierung oder Sofortpigmentierung verantwortlich.

Strahlungsquellen zur Bräunung der Haut werden im privaten und gewerblichen Umfeld eingesetzt. In beiden Fällen gelten für den Betrieb strenge Auflagen, bei deren Beachtung alle für den Gesundheitsschutz geltenden Grenzwerte eingehalten werden. Zum Beispiel kann in Abhängigkeit des Hauttyps eine maximale Bestrahlungsdauer so festgelegt werden, dass kein UV-Erythem entsteht. Auch für die z.B. im Laufe eines Jahres erhaltene Bestrahlungsdosis können Grenzwerte festgelegt und überwacht werden, zum Beispiel um eine signifikante Erhöhung des Risikos, an weißem Hautkrebs zu erkranken, auszuschließen. Somit können alle erwünschten und unerwünschten Wirkungen der Bestrahlung sehr gut kontrolliert und überwacht werden, in der Regel besser als bei einer Bestrahlung der Haut durch die Sonne. Darum gilt die Bräunung der Haut in einem Solarium heutzutage als sehr sicher.

In der Druckschrift "Schutz des Menschen vor den Gefahren solarer UV-Strahlung und UV-Strahlung in Solarien", Empfehlung der Strahlenschutzkommission Bonn, von Beate Volkmer et al., verabschiedet in der 280. Sitzung der Strahlenschutzkommission am 11./12. Februar 2016, XP55852193A, sind die skizzierten Grundlagen der photobiologischen Wirksamkeit der UV-Strahlung beschrieben.

Aus der Druckschrift US 2013/0172963 A1 ist eine UVB-Strahlungsquelle bekannt geworden, die nicht zur Bräunung der Haut, sondern zur Förderung der Vitamin-D-Synthese eingesetzt werden soll. Sie sendet schmalbandig UV-Strahlung in einem Wellenlängenbereich um 297 nm herum aus, insbesondere im Bereich von 292 nm bis 302 nm.

Aus der Druckschrift FR 2 764 813 A1 ist eine faseroptische Lichtabgabeausrüstung für kosmetische und phototherapeutische Anwendungen bekannt geworden, bei der Lichtleiter das Licht bis nahe an die Haut eines Patienten heranführen. Eine großflächige Bestrahlung zu Bräunungszwecken ist damit nicht möglich, weil die abgegebene Leistung viel zu gering ist.

Davon ausgehend ist es die Aufgabe der Erfindung, ein Solarium zur Verfügung zu stellen, die bzw. das bei ausreichender Bräunungswirkung ein nochmals verringertes NMSC-Krebsrisiko darstellt.

Die Aufgabe wird gelöst durch das Solarium mit den Merkmalen des Anspruchs 1. Vorteilhafte Ausgestaltungen sind in den Unteransprüchen angegeben.

Die Strahlungsquelle ist für ein Hautbestrahlungsgerät zur Bräunung der Haut bestimmt und zur Ausstrahlung von UV-Licht mit einer spektralen Bestrahlungsstärke $E_\lambda(\lambda)$ ausgebildet, wobei die spektrale Bestrahlungsstärke $E_\lambda(\lambda)$ so gewählt ist, dass das Verhältnis der NMSC-wirksamen, biologischen Bestrahlungsstärke $E_{NMSC}$ zur erythemwirksamen, biologischen Bestrahlungsstärke $E_{er}$ 0,9 oder weniger beträgt.

Die Strahlungsquelle ist dazu ausgebildet, UV-Licht in einem Wellenlängenbereich von 280 nm bis 400 nm oder in Teilen davon auszustrahlen. Zusätzlich kann Strahlung in anderen Wellenlängenbereichen ausgesendet werden, insbesondere sichtbares Licht. Das ausgesendete Wellenlängenspektrum entspricht einer spektralen Bestrahlungsstärke $E_\lambda(\lambda)$. Diese Bestrahlungsstärke beschreibt die eingestrahlte Leistung pro Fläche und kann in W/m$^2$ angegeben werden. Sie hängt vom Abstand zwischen der Strahlungsquelle und der bestrahlten Fläche ab. Dieser Abstand wird in der Praxis von den konstruktiven Gegebenheiten eines Solariums vorgegeben und entspricht insbesondere dem Abstand zwischen der Strahlungsquelle und einer zu bestrahlenden Hautpartie. Die spektrale Bestrahlungsstärke $E_\lambda(\lambda)$ kann grundsätzlich eine beliebige Verteilung aufweisen, zum Beispiel ein weitgehend kontinuierliches Spektrum, dass sich über den gesamten UV-Bereich erstreckt oder eine Kombination einzelner mehr oder weniger ausgeprägter Peaks. Insbesondere kann die spektrale Bestrahlungsstärke $E_\lambda(\lambda)$ einen Maximalwert, d.h. ein globales Maximum oder ein lokales Maximum aufweisen, dass in einem Wellenlängenbereich von 330 nm bis 390 nm liegt.

Wie eingangs erläutert können aus dieser spektralen Bestrahlungsstärke $E_\lambda(\lambda)$ die NMSC-wirksame, biologische Bestrahlungsstärke $E_{NMSC}$ und die erythemwirksame, biologische Bestrahlungsstärke $E_{er}$ berechnet werden. Die hierfür heranzuziehenden spektralen Wirkungsfaktoren sind in Fachkreisen allgemein bekannt und können bei Bedarf der eingangs zitierten Norm DIN 5031-10:2018-03 entnommen werden, insbesondere der darin enthaltenen Tabelle A.3, die Angaben zum spektralen Wirkungsfaktor $A_{er}(\lambda)$ für das UV-Erythem enthält, und der Tabelle A.6, die Angaben zum spektralen Wirkungsfaktor $A_{nmsc}(\lambda)$ für die NMSC-Photokarzinogenese enthält.

Eine Besonderheit der Erfindung besteht darin, dass das Wellenlängenspektrum der Strahlungsquelle und damit die spektrale Bestrahlungsstärke $E_\lambda(\lambda)$ der Strahlungsquelle so gewählt ist, dass das Verhältnis zwischen $E_{NMSC}$ und $E_{er}$ 0,9 oder weniger beträgt. Wahlweise kann das Verhältnis auch noch kleiner gehalten werden und beispielsweise 0,8 oder weniger, 0,7 oder weniger, 0,6 oder weniger oder 0,5 oder weniger betragen. Für diese Wahl der spektralen Bestrahlungsstärke $E_\lambda(\lambda)$ stehen dem Fachmann unterschiedliche Maßnahmen zur Verfügung. Grundsätzlich kann die spektrale Bestrahlungsstärke $E_\lambda(\lambda)$ dadurch beeinflusst werden, dass die Entstehung der UV-Strahlung auf bestimmte Wellenlängenbereiche konzentriert wird. Die hierzu zur Verfügung stehenden Maßnahmen sind vom Typ der Strahlungsquelle abhängig und werden weiter unten näher erläutert. Alternativ können Anteile der erzeugten Strahlung herausgefiltert werden, z.B. durch Anordnung eines entsprechenden Filters zwischen dem Ort der Strahlungserzeugung und der Haut. Hierzu geeignet sind z.B. Filterscheiben, die im Zusammenhang mit UV-Hochdruckbrennern eingesetzt werden, aber auch spezielle Glas- oder Kunststoffmaterialien, die als Teil des Gehäuses der Strahlungsquelle verbaut werden können.

Der tatsächliche Zusammenhang zwischen der NMSC-wirksamen, biologischen Bestrahlungsstärke und dem Risiko eines dieser Bestrahlung ausgesetzten Menschen, an weißem Hautkrebs zu erkranken, ist wissenschaftlich noch nicht exakt bekannt. Die veröffentlichten Wirkungsfaktoren beruhen auf Daten aus Experimenten an Mäusen, die auf die UV-Transmission der menschlichen Haut hochgerechnet und extrapoliert wurden. Es ist jedoch unbestritten, dass eine höhere NMSC-wirksame Bestrahlungsstärke mit einem höheren Risiko einhergeht.

In diesem Zusammenhang hat der Erfinder erkannt, dass die Wirkungsspektren für das UV-Erythem und für die NMSC-Photokarzinogenese einander zwar stark überlappen, sich jedoch hinreichend voneinander unterschieden, sodass es bei geeigneter Wahl der spektralen Bestrahlungsstärke $E_\lambda(\lambda)$ der Strahlungsquelle möglich ist, die NMSC-wirksame Bestrahlungsstärke immer spürbar niedriger zu halten, als die erythemwirksame Bestrahlungsstärke. Dies führt dazu, dass sich eine zu hohe Bestrahlungsdosis wegen der relativ hohen Erythemwirksamkeit in Form eines Sonnenbrands bemerkbar machen wird, bevor ein hohes NMSC-Risiko entsteht. Bei maßvoller Bestrahlung, die nicht zu einem UV-Erythem führt, bleibt auch die NMSC-wirksame, biologische Bestrahlung relativ gering. Im Ergebnis kann das NMSC-Risiko durch die gezielte Wahl der spektralen Bestrahlungsstärke $E_\lambda(\lambda)$ der Strahlungsquelle also besonders gering gehalten werden. Dies gilt sowohl im Vergleich zu einer Bestrahlung mit Sonnenlicht, die typischerweise ein Verhältnis zwischen $E_{NMSC}$ und $E_{er}$ von etwa 2,2 aufweist, als auch im Vergleich zu handelsüblichen Bestrahlungsgeräten, bei denen der Erfinder je nach Bauart Verhältnisse zwischen $E_{NMSC}$ und $E_{er}$ von mindestens 1,1, im Regelfall aber 1,4 oder mehr, ermittelt hat.

Ein weiterer Vorteil ist, dass in der Praxis die Bestrahlungsdosis stets auf Grundlage der erythemwirksamen Bestrahlungsstärke vorgegeben wird. Zu diesem Zweck sind die Strahlungsquellen, in diesem Fall Niederdruckröhren, mit einem sogenannten UV-Schlüssel versehen, der zwei als X und Y bezeichnete Zahlenwerte umfasst. Der X-Wert gibt die erythemwirksame Bestrahlungsstärke bei freibrennender Lampe in einem Abstand von 25 cm in mW/m² an. Unter Berücksichtigung des tatsächlichen Abstands zwischen Haut und Strahlungsquelle und des jeweiligen Hauttyps wird die maximale Bestrahlungsdauer festgelegt. Bei diesem Vorgehen wird durch die Erfindung sichergestellt, dass ohne weiteres Zutun die NMSC-wirksame Bestrahlungsstärke ebenfalls gering gehalten wird.

In einer Ausgestaltung weist die spektrale Bestrahlungsstärke $E_\lambda(\lambda)$ im Wellenlängenbereich von 330 nm bis 390 nm einen Maximalwert auf und im Wellenlängenbereich von 299 nm bis 311 nm und im Wellenlängenbereich von 315 nm bis 329 nm Werte, die 5 % dieses Maximalwerts nicht überschreiten. Wahlweise kann der Wellenlängenbereich, in dem die Grenze von 5 % des Maximalwerts nicht überschritten wird, auch bereits bei 296 nm beginnen, also die Bereiche von 296 nm bis 311 nm und von 315 nm bis 329 nm umfassen. Die Grenze von 5 % kann wahlweise auch niedriger gewählt werden, beispielsweise bei 3,5 % des Maximalwerts, bei 2 % des Maximalwerts oder sogar bei 1 % des Maximalwerts. Diese Begrenzungen der spektralen Bestrahlungsstärke $E_\lambda(\lambda)$ tragen dazu bei, das Verhältnis zwischen $E_{NMSC}$ und $E_{er}$ gering zu halten, weil der spektrale Wirkungsfaktor $A_{NMSC}(\lambda)$ für die NMSC-Photokarzinogenese in den genannten Wellenlängenbereichen größer ist, als der spektrale Wirkungsfaktor $A_{er}(\lambda)$ für das UV-Erythem. Der in dieser Ausgestaltung von der Beschränkung ausgenommene Wellenlängenbereich zwischen 311 nm und 315 nm weist zwar ebenfalls ein ungünstiges Verhältnis zwischen den beiden spektralen Wirkungsfaktoren auf, weist jedoch bei einigen Strahlungsquellen, deren Emissionsspektrum einen Quecksilberpeak bei 313 nm hat, eine höhere spektrale Bestrahlungsstärke $E_\lambda(\lambda)$ auf, deren Unterdrückung zusätzliche Maßnahmen erfordern würde und akzeptiert werden kann, solange das gewünschte Verhältnis zwischen $E_{NMSC}$ und $E_{er}$ eingehalten wird.

In einer Ausgestaltung weist die spektrale Bestrahlungsstärke $E_\lambda(\lambda)$ im Wellenlängenbereich von 330 nm bis 390 nm einen Maximalwert und im Wellenlängenbereich von 299 nm bis 329 nm Werte auf, die 5 % dieses Maximalwerts nicht überschreiten. Auch in dieser Ausgestaltung kann der Wellenlängenbereich, in dem die Grenze von 5 % des Maximalwerts nicht überschritten wird, alternativ bereits bei 296 nm beginnen, also den Bereich von 296 nm bis 329 nm umfassen. Die Grenze von 5 % kann wahlweise auch niedriger gewählt werden, beispielsweise bei 3,5 % des Maximalwerts, bei 2 % des Maximalwerts oder sogar bei 1 % des Maximalwerts. Diese Begrenzungen der spektralen Bestrahlungsstärke $E_\lambda(\lambda)$ tragen ebenfalls dazu bei, das Verhältnis zwischen $E_{NMSC}$ und $E_{er}$ gering zu halten, wobei der Bereich eines Quecksilberpeaks einbezogen ist.

In einer Ausgestaltung weist die spektrale Bestrahlungsstärke $E_\lambda(\lambda)$ im Wellenlängenbereich von 330 nm bis 364 nm einen Maximalwert und für Wellenlängen größer als 364 nm Werte auf, die 5 % dieses Maximalwerts nicht überschreiten. Die Grenze von 5 % kann wahlweise auch niedriger gewählt werden, beispielsweise bei 3,5 % des Maximalwerts, bei 2 % des Maximalwerts oder sogar bei 1 % des Maximalwerts. Durch diese Begrenzungen der spektralen Bestrahlungsstärke $E_\lambda(\lambda)$ wird ein weiterer Wellenlängenbereich ausgeklammert, in dem das Verhältnis zwischen $E_{NMSC}$ und $E_{er}$ ungünstig ist.

Grundsätzlich kann die Strahlungsquelle beliebige Typen und Anzahlen von Strahlungskörpern aufweisen, einschließlich Kombinationen unterschiedlicher Typen. In einer Ausgestaltung weist die Strahlungsquelle eine UV-Niederdrucklampe auf. Durch Zusammenstellung des enthaltenen Leuchtstoffs kann das Spektrum der entstehenden Strahlung beeinflusst werden. Die Leuchtstoffzusammenstellung kann z.B. Phosphor in unterschiedlichen chemischen Verbindungen enthalten. Ebenfalls kann ein Glas der UV-Niederdrucklampe, das die Leuchtstoffzusammensetzung umschließt, als Filter eingesetzt werden, zum Beispiel durch Aufbringen einer geeigneten Beschichtung. Es ist daher unter Rückgriff auf die bewährte Technologie von Leuchtstofflampen ohne weiteres möglich, das erwünschte Verhältnis zwischen $E_{NMSC}$ und $E_{er}$ zu erzielen.

In einer Ausgestaltung weist die Strahlungsquelle einen UV-Hochdruckbrenner auf. Bei UV-Hochdruckbrennern ergibt sich das ausgesendete Spektrum ebenfalls aus den Eigenschaften des Leuchtmediums, welche durch eine Dotierung beeinflusst werden können, in der Regel in Kombination mit einer Filterscheibe. Es ist daher ähnlich wie bei einer UV-Niederdrucklampe ohne weiteres möglich, die spektrale Bestrahlungsstärke $E_\lambda(\lambda)$ so zu wählen, dass das gewünschte Verhältnis zwischen $E_{NMSC}$ und $E_{er}$ erreicht wird. Ebenfalls kann ein Glas der UV-Niederdrucklampe, das die Leuchtstoffzusammensetzung umschließt, als Filter eingesetzt werden, da die Zusammensetzung des Glases eine differenzierte Emission der Strahlung zulässt, so dass in Kombination mit der Leuchtstoffzusammensetzung das gewünschte Verhältnis zwischen $E_{NMSC}$ und $E_{er}$ erzielt

wird. Hierzu kann bewährte Technologie eingesetzt werden.

In einer Ausgestaltung weist die Strahlungsquelle eine UV-LED auf. LED steht für *light emitting diode,* Leuchtdiode. Bei UV-LEDs entsteht die UV-Strahlung durch Rekombination von Elektronen und Löchern infolge von Übergängen von Elektronen aus dem Leitungsband in das Valenzband. Die Bandlücke zwischen Leitungs- und Valenzband bestimmt die Wellenlänge. Durch Auswahl und Dotierung der verwendeten Halbleitermaterialien können die Breite von Leitungs- und Valenzband und deren Abstand gezielt beeinflusst werden. Natürlich kann die Strahlungsquelle eine Vielzahl von UV-LEDs aufweisen, insbesondere eine Vielzahl von UV-LEDs mit unterschiedlichen Wellenlängen. Somit kann auch unter Verwendung von UV-LEDs die spektrale Bestrahlungsstärke $E_\lambda(\lambda)$ so gewählt werden, dass das gewünschte Verhältnis zwischen $E_{NMSC}$ und $E_{er}$ erreicht wird.

In einer Ausgestaltung weist die Strahlungsquelle eine Filterscheibe auf. Dadurch können gezielt bestimmte Wellenlängenbereiche aus der erzeugten Strahlung herausgefiltert werden. Das Material der Filterscheibe kann auf den herauszufilternden Wellenlängenbereich abgestimmt werden und es können auch mehrere Filterscheiben kombiniert werden. Die Ausführung mit einer Filterscheibe ist besonders in Kombination mit einem UV-Hockdruckbrenner sinnvoll, jedoch nicht darauf beschränkt.

Bei der Erfindung beträgt die biologisch wirksame Bestrahlungsstärke für die direkte Pigmentierung $E_{pi}$ mindestens 180 W/m$^2$, bevorzugt mindestens 200 W/m$^2$. Diese Angabe bezieht sich auf einen Abstand zwischen der Haut und der Strahlungsquelle, der im Betrieb des Hautbestrahlungsgerät eingehalten wird. Durch die angegebene Bestrahlungsstärke wird eine gute Bräunungswirkung erzielt.

In einer Ausgestaltung überschreitet die erythemwirksame, biologisch wirksame Bestrahlungsstärke $E_{er}$ 1,0 W/m$^2$, bevorzugt 0,3 W/m$^2$ nicht. Auch diese Angabe bezieht sich auf einen Abstand zwischen der Haut und der Strahlungsquelle, der im Betrieb des Hautbestrahlungsgerät eingehalten wird. Eine zu starke Bestrahlung wird vermieden.

In einer Ausgestaltung ist das Solarium eine Sonnenbank oder eine Sonnendusche.

Nachfolgend wird die Erfindung anhand von in Figuren dargestellten Ausführungsbeispielen näher erläutert. Es zeigen:

Fig. 1   ein Diagramm mit den relativen spektralen Empfindlichkeiten für das UV-Erythem und für NMSC,

Fig. 2   ein Diagramm der spektralen Bestrahlungsstärke $E_\lambda(\lambda)$ eines LED-Schulterbräuners nach dem Stand der Technik,

Fig. 3   ein Diagramm der spektralen Bestrahlungsstärke $E_\lambda(\lambda)$ eines Hochdruckstrahlers mit Filterscheibe nach dem Stand der Technik,

Fig. 4   ein Diagramm der spektralen Bestrahlungsstärke $E_\lambda(\lambda)$ von UV-Niederdrucklampen nach dem Stand der Technik,

Fig. 5   ein Diagramm der spektralen Bestrahlungsstärke $E_\lambda(\lambda)$ einer erfindungsgemäßen UV-LED.

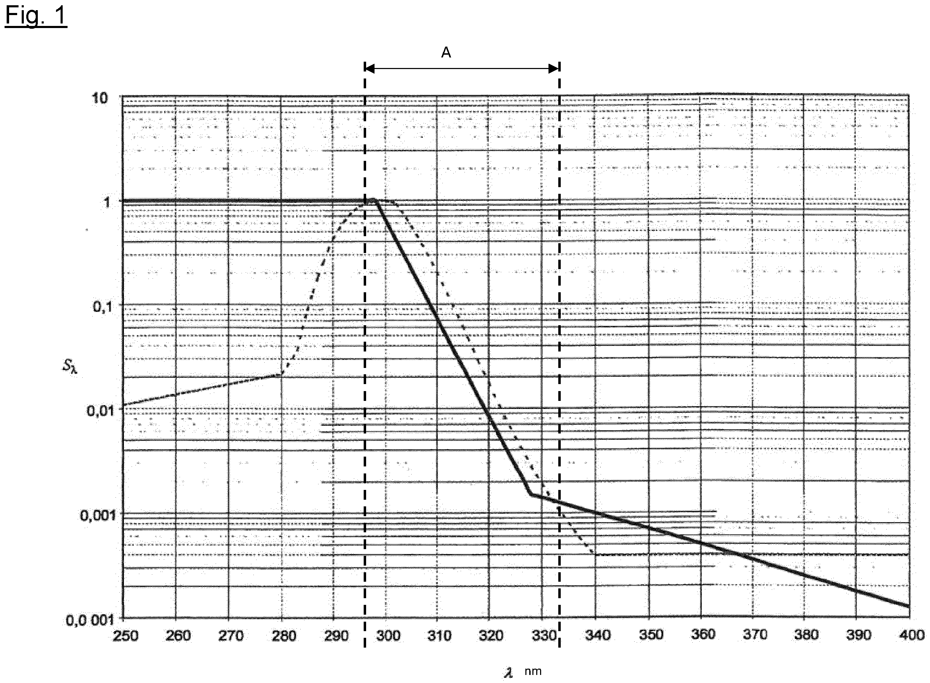

In Fig. 1 sind die relativen spektralen Empfindlichkeiten $S\lambda$ für das UV-Erythem und für NMSC aufgetragen über die Wellenlänge $\lambda$ in Nanometern. Für dieselben Größen wurde vorstehend die Bezeichnung $A_{biol}(\lambda)$ verwendet. Die relative spektrale Empfindlichkeit $S_\lambda$ ist normiert und einheitenlos und in Figur 1 logarithmisch aufgetragen. Die gestrichelte Kurve zeigt die relative spektrale Empfindlichkeit für NMSC, die durchgezogene Linie diejenige für das UV-Erythem. Es ist ersichtlich, dass insbesondere in dem mit A gekennzeichneten Wellenlängenbereich zwischen ca. 296 nm und 333 nm die Wirkungsfunktionen für das Erythem sowie für NMSC sehr nah beieinanderliegen, NMSC das Erythem sogar übersteigt. Die beiden Wirkungsfunktionen sind in der Norm DIN 5031-10:2018-03 in Tabellenform angegeben.

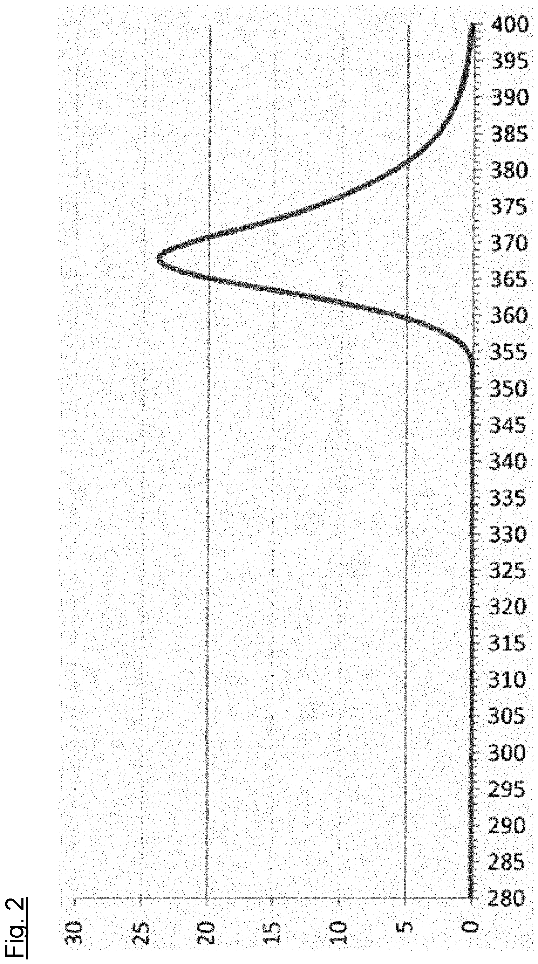

Die Figuren 2 bis 5 zeigen jeweils ein Diagramm einer spektralen Bestrahlungsstärke $E_\lambda(\lambda)$ einer UV-Strahlungsquelle, angegeben bzw. gemessen im bei der Bräunung der Haut vorgesehenen Abstand. Aufgetragen ist die in einem Wellenlängenintervall $\Delta\lambda$ von 1 nm auftreffende Leistung in W/m$^2$ über die Wellenlänge $\lambda$ in nm. Auf dieser Grundlage wurden für jede der UV-Strahlungsquellen die erythemwirksame Bestrahlungsstärke $E_{er}$ und die NMSC-wirksame Bestrahlungsstärke $E_{NMSC}$ berechnet, durch Bilden der folgenden Summe:

$$E_{\text{eff}} = \sum_{250\text{ nm}}^{400\text{ nm}} S_\lambda E_\lambda \varDelta_\lambda$$

Demnach wurde wie in der genannten Norm vorgegeben die jeweilige spektrale Empfindlichkeit mit der spektralen Bestrahlungsstärke für jedes Wellenlängenintervall multipliziert und die Produkte wurden aufsummiert.

Die spektrale Bestrahlungsstärke $E_\lambda(\lambda)$ aus Fig. 2 wurde mit einem Doppelmonochromator an einem LED-Schulterbräuner der Marke Blue Vision gemessen. Die ermittelte NMSC-wirksame Bestrahlungsstärke $E_{NMSC}$ beträgt 0,139 W/m$^2$, die erythemwirksame Bestrahlungsstärke $E_{er}$ beträgt 0,126 W/m$^2$. Das Verhältnis $E_{NMSC}$ zu $E_{er}$ beträgt 1, 10.

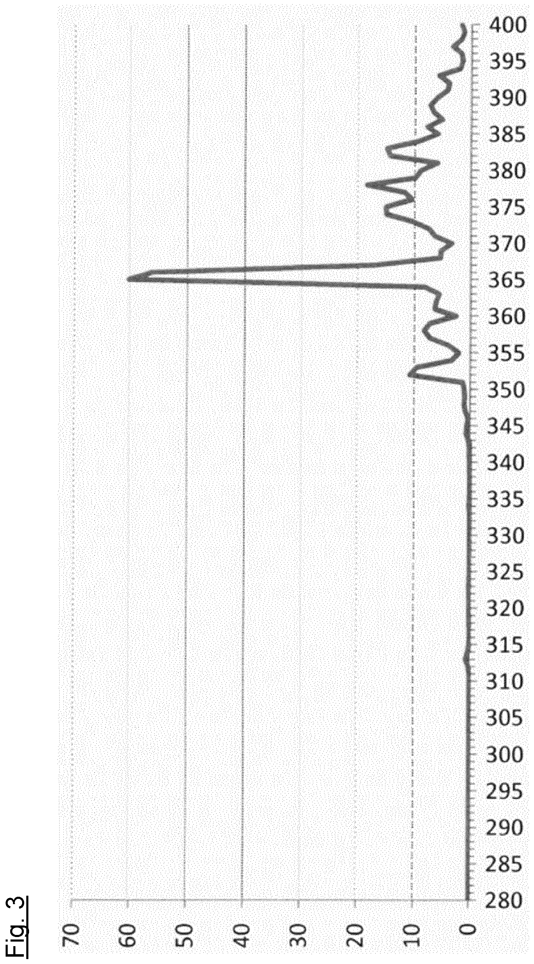

Die spektrale Bestrahlungsstärke $E_\lambda(\lambda)$ aus Fig. 3 wurde mit einem Doppelmonochromator an einem UV-Hochdruckstrahler mit Filterscheibe (Prestige 1400, Filterscheibe Typ 3) gemessen. Die ermittelte NMSC-wirksame Bestrahlungsstärke $E_{NMSC}$ beträgt 0,464 W/m², die erythemwirksame Bestrahlungsstärke $E_{er}$ beträgt 0,299 W/m². Das Verhältnis $E_{NMSC}$ zu $E_{er}$ beträgt 1,55

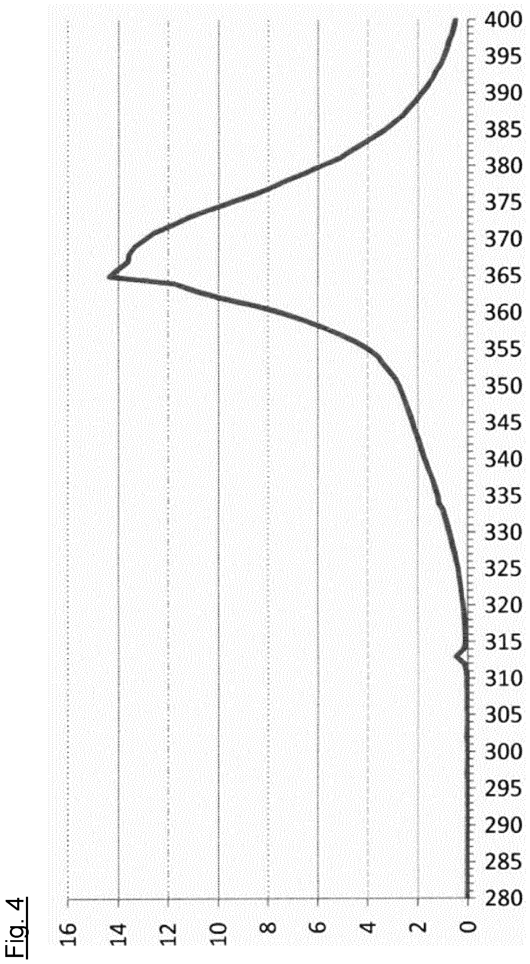

Die spektrale Bestrahlungsstärke $E_\lambda(\lambda)$ aus Fig. 4 wurde mit einem Doppelmonochromator an UV-Niederdrucklampen (Ergo 1400 New Technology x-Tend plus mit Oberteil) gemessen. Die ermittelte NMSC-wirksame Bestrahlungsstärke $E_{NMSC}$ beträgt 0,408 W/m², die erythemwirksame Bestrahlungsstärke $E_{er}$ beträgt 0,291 W/m². Das Verhältnis $E_{NMSC}$ zu $E_{er}$ beträgt 1,40.

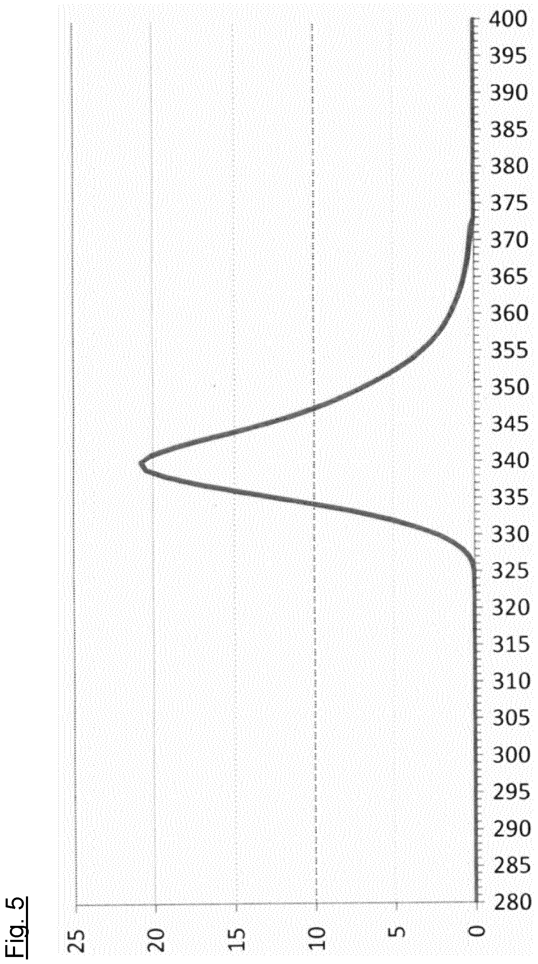

Fig. 5 zeigt die spektrale Bestrahlungsstärke $E_\lambda(\lambda)$ einer erfindungsgemäßen Strahlungsquelle, die eine UV-LED aufweist. Die ausgesendete Strahlung erstreckt sich über einen Wellenlängenbereich von etwa 325 nm bis etwa 372 nm und weist einen Maximalwert von 20,8 W/m² bei 340 nm auf. Die ermittelte NMSC-wirksame Bestrahlungsstärke $E_{NMSC}$ beträgt 0,170 W/m², die erythemwirksame Bestrahlungsstärke $E_{er}$ beträgt 0,292 W/m². Das Verhältnis $E_{NMSC}$ zu $E_{er}$ beträgt 0,59.

**Patentansprüche**

1. Solarium zur Bräunung der Haut mit einer Strahlungsquelle, die zur Ausstrahlung von UV-Licht mit einer spektralen Bestrahlungsstärke $E_\lambda(\lambda)$ ausgebildet ist, wobei die biologisch wirksame Bestrahlungsstärke für die direkte Pigmentierung $E_{pi}$ mindestens 180 W/m² beträgt, **dadurch gekennzeichnet, dass** die spektrale Bestrahlungsstärke $E_\lambda(\lambda)$ so gewählt ist, dass das Verhältnis der NMSC-wirksamen, biologischen Bestrahlungsstärke $E_{NMSC}$ zur erythemwirksamen, biologischen Bestrahlungsstärke $E_{er}$ 0,9 oder weniger beträgt.

2. Solarium nach Anspruch 1, **dadurch gekennzeichnet, dass** die spektrale Bestrahlungsstärke $E_\lambda(\lambda)$ im Wellenlängenbereich von 330 nm bis 390 nm einen Maximalwert aufweist und im Wellenlängenbereich von 299 nm bis 311 nm und im Wellenlängenbereich von 315 nm bis 329 nm Werte aufweist, die 5 % dieses Maximalwerts nicht überschreiten.

3. Solarium nach Anspruch 1, **dadurch gekennzeichnet, dass** die spektrale Bestrahlungsstärke $E_\lambda(\lambda)$ im Wellenlängenbereich von 330 nm bis 390 nm einen Maximalwert aufweist und im Wellenlängenbereich von 299 nm bis 329 nm Werte aufweist, die 5 % dieses Maximalwerts nicht überschreiten.

4. Solarium nach einem der Ansprüche 1 bis 3, **dadurch gekennzeichnet, dass** die spektrale Bestrahlungsstärke $E_\lambda(\lambda)$ im Wellenlängenbereich von 330 nm bis 364 nm einen Maximalwert aufweist und für Wellenlängen größer als 364 nm Werte aufweist, die 5 % dieses Maximalwerts nicht überschreiten.

5. Solarium nach einem der Ansprüche 1 bis 4, **dadurch gekennzeichnet, dass** die Strahlungsquelle eine UV-Niederdrucklampe aufweist.

6. Solarium nach einem der Ansprüche 1 bis 5, **dadurch gekennzeichnet, dass** die Strahlungsquelle einen UV-Hochdruckbrenner aufweist.

7. Solarium nach einem der Ansprüche 1 bis 6, **dadurch gekennzeichnet, dass**, die Strahlungsquelle eine UV-LED aufweist.

8. Solarium nach einem der Ansprüche 1 bis 7, **dadurch gekennzeichnet, dass**, die Strahlungsquelle eine Filterscheibe aufweist.

9. Solarium nach einem der Ansprüche 1 bis 8, **dadurch gekennzeichnet, dass** die biologisch wirksame Bestrahlungsstärke für die direkte Pigmentierung $E_{pi}$ mindestens 200 W/m² beträgt.

10. Solarium nach einem der Ansprüche 1 bis 9, **dadurch gekennzeichnet, dass** die erythemwirksame, biologisch wirksame Bestrahlungsstärke $E_{er}$ 1,0 W/m², bevorzugt 0,3 W/m² nicht überschreitet.

11. Solarium nach einem der Ansprüche 1 bis 10, **dadurch gekennzeichnet, dass** das Solarium eine Sonnenbank oder eine Sonnendusche ist.

**Claims**

1. A solarium for tanning skin with a radiation source, designed to emit UV light at a spectral irradiance $E_\lambda(\lambda)$, the biologically effective irradiance for the direct pigmentation $E_{pi}$ being at least 180 W/m², **characterized in that** the spectral irradiance $E_\lambda(\lambda)$ is chosen such that the ratio of the NMSC-effective, biological irradiance $E_{NMSC}$ to the erythema-effective, biological irradiance $E_{er}$ is 0.9 or less.

2. The solarium according to Claim 1, **characterized in that** the spectral irradiance $E_\lambda(\lambda)$ has a maximum value in the wavelength range from 330 nm to 390 nm, and has values in the wavelength range from 299 nm to 311 nm and in the wavelength range from 315 nm to 329 nm, which do not exceed 5% of said maximum value.

3. The solarium according to Claim 1, **characterized in that** the spectral irradiance $E_\lambda(\lambda)$ has a maximum value in the wavelength range from 330 nm to 390 nm, and has values in the wavelength range from 299 nm to 329 nm, which do not exceed 5% of said maximum value.

**4.** The solarium according to any one of Claims 1 to 3, **characterized in that** the spectral irradiance $E_\lambda(\lambda)$ has a maximum value in the wavelength range from 330 nm to 364 nm, and has values for wavelengths greater than 364 nm, which do not exceed 5% of said maximum value.

**5.** The solarium according to any one of Claims 1 to 4, **characterized in that** the radiation source has a low-pressure UV lamp.

**6.** The solarium according to any one of Claims 1 to 5, **characterized in that** the radiation source has a high-pressure UV burner.

**7.** The solarium according to any one of Claims 1 to 6, **characterized in that** the radiation source has a UV LED.

**8.** The solarium according to any one of Claims 1 to 7, **characterized in that** the radiation source has a filter disk.

**9.** The solarium according to any one of Claims 1 to 8, **characterized in that** the biologically effective irradiance for the direct pigmentation $E_{pi}$ is at least 200 W/m$^2$.

**10.** The solarium according to any one of Claims 1 to 9, **characterized in that** the erythema-effective, biologically effective irradiance $E_{er}$ does not exceed 1.0 W/m$^2$, preferably does not exceed 0.3 W/m$^2$.

**11.** The solarium according to any one of Claims 1 to 10, **characterized in that** the solarium is a sunbed or a sun shower.

**Revendications**

**1.** Solarium destiné au bronzage de la peau avec une source de rayonnement conçue pour l'émission de lumière UV avec une intensité d'irradiation spectrale $E_\lambda(\lambda)$, dans lequel l'intensité d'irradiation biologiquement active pour la pigmentation directe $E_{pi}$ mesure au moins 180 W/m$^2$, **caractérisé en ce que** l'intensité d'irradiation spectrale $E_\lambda(\lambda)$ est sélectionnée de telle façon que le rapport de l'intensité d'irradiation biologique à action NMSC $E_{NMSC}$ à l'intensité d'irradiation biologique à action éryhtémateuse $E_{er}$ s'élève à 0,9 ou moins.

**2.** Solarium selon la revendication 1, **caractérisé en ce que** l'intensité d'irradiation spectrale $E_\lambda(\lambda)$ présente une valeur maximale dans la plage de longueur d'onde de 330 nm à 390 nm et présente des valeurs ne dépassant pas 5 % de cette valeur maximale dans la plage de longueur d'onde de 299 nm à 311 nm et dans la plage de longueur d'onde de 315 nm à 329 nm.

**3.** Solarium selon la revendication 1, **caractérisé en ce que** l'intensité d'irradiation spectrale $E_\lambda(\lambda)$ présente une valeur maximale dans la plage de longueur d'onde de 330 nm à 390 nm et présente des valeurs ne dépassant pas 5 % de cette valeur maximale dans la plage de longueur d'onde de 299 nm à 329 nm.

**4.** Solarium selon l'une des revendications 1 à 3, **caractérisé en ce que** l'intensité d'irradiation spectrale $E_\lambda(\lambda)$ présente une valeur maximale dans la plage de longueur d'onde de 330 nm à 364 nm et présente des valeurs ne dépassant pas 5 % de cette valeur maximale pour des longueurs d'onde supérieures à 364 nm.

**5.** Solarium selon l'une des revendications 1 à 4, **caractérisé en ce que** la source de rayonnement présente une lampe basse pression UV.

**6.** Solarium selon l'une des revendications 1 à 5, **caractérisé en ce que** la source de rayonnement présente un brûleur haute pression UV.

**7.** Solarium selon l'une des revendications 1 à 6, **caractérisé en ce que** la source de rayonnement présente une DEL UV.

**8.** Solarium selon l'une des revendications 1 à 7, **caractérisé en ce que** la source de rayonnement présente une vitre filtrante.

**9.** Solarium selon l'une des revendications 1 à 8, **caractérisé en ce que** l'intensité d'irradiation biologiquement active pour la pigmentation directe $E_{pi}$ mesure au moins 200 W/m$^2$.

**10.** Solarium selon l'une des revendications 1 à 9, **caractérisé en ce que** l'intensité d'irradiation biologique à action éryhtémateuse $E_{er}$ ne dépasse pas 1,0 W/m$^2$, de préférence pas 0,3 W/m$^2$.

**11.** Solarium selon l'une des revendications 1 à 10, **caractérisé en ce que** le solarium est un banc solaire ou une douche solaire.

Fig. 1

EP 4 337 309 B1

Fig. 2

EP 4 337 309 B1

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20130172963 A1

- FR 2764813 A1

**In der Beschreibung aufgeführte Nicht-Patentliteratur**

- **VON BEATE VOLKMER.** Schutz des Menschen vor den Gefahren solarer UV-Strahlung und UV-Strahlung in Solarien. *Empfehlung der Strahlenschutzkommission Bonn*